# FASCICULE DE BREVET EUROPEEN

(11) **EP 1 721 142 B1**
(45) Date de publication et mention de la délivrance du brevet: **23.05.2007**
(21) Numéro de dépôt: 05717700.8
(22) Date de dépôt: 02.02.2005
(51) Int. Cl.: G01N 3/56, G01N 33/30

(54) **TRIBOMETRE**
TRIBOMETER
TRIBOMETER

(30) Priorité: 03.02.2004 FR 0401017
(43) Date de publication de la demande: 15.11.2006
(73) Titulaire: CENTRE NATIONAL DE LA RECHERCHE SCIENTIFIQUE (CNRS), 75794 Paris Cedex 16 (FR); ECOLE CENTRALE DE LYON, 69134 Ecully Cedex (FR)
(72) Inventeur: MAZUYER, Denis, F-69130 ECULLY (FR); LARGE, André, F-69001 LYON (FR)
(74) Mandataire: Ramey, Daniel
(86) Numéro de dépôt international: PCT/FR2005/050063
(87) Numéro de publication internationale: WO 2005/075956

(56) Documents cités:
- FR-A- 1 500 941
- FR-A- 2 036 123
- US-A- 2 106 170
- US-A- 5 955 655
- US-A- 6 145 370
- PATENT ABSTRACTS OF JAPAN vol. 0102, no. 82 (P-500), 25 septembre 1986 (1986-09-25) & JP 61 102539 A (KUNIO TSUJI), 21 mai 1986 (1986-05-21)

## Description

L'invention concerne un tribomètre permettant notamment de générer des pressions de contact et/ou des vitesses de frottement de glissement très élevées.

Dans certains domaines de production, comme la mise en forme des métaux, les gains de productivité et la réduction des coûts de maintenance imposent des contacts entre surfaces frottantes, lubrifiés ou non, soumis à des conditions de fonctionnement de plus en plus sévères. On peut citer, à titre d'exemple, le procédé de tréfilage de fils d'acier laitonné dans la fabrication de carcasses de pneumatiques à structure radiale, procédé qui reste l'un des plus complexes en termes de lubrification. Les aciers, dont la limite élastique peut être de plus en plus élevée, sont transformés à des vitesses de plus en plus importantes. De nouvelles formulations de lubrifiant plus performantes doivent être envisagées pour permettre la maîtrise du tréfilage d'aciers durs, mais aussi d'autres métaux, à des vitesses extrêmement élevées.

Par ailleurs, afin de prédire la durée de vie d'un système mécanique (moteur, transmission, roulement), il est nécessaire d'accélérer le processus de vieillissement des surfaces frottantes en accroissant la sévérité des conditions tribologiques auxquelles ces surfaces sont soumises, en accroissant notamment la cinétique d'usure des surfaces. Ceci peut permettre de réduire les essais lourds et coûteux sur bancs industriels.

Trois paramètres sont particulièrement cruciaux pour la compréhension des phénomènes d'usure et des mécanismes de lubrification à hautes pressions et hautes vitesses :
- la température de contact, qui peut être supérieure à 500 °C, et qui peut limiter les performances actuelles des lubrifiants,
- le temps de contact, qui peut être très court (de l'ordre de quelques µs) si les vitesses de glissement sont très élevées (supérieure à 1 m/s),
- le gradient de pression subi par le lubrifiant, lors de son passage dans le contact entre les surfaces frottantes.

De manière classique, le comportement tribologique des surfaces et des lubrifiants est évalué d'abord pour des pressions et vitesses peu élevées, accessibles sur des tribomètres conventionnels tels que ceux commercialisés par les sociétés FALEX et PLINT & PARTNERS, puis les résultats obtenus sont extrapolés pour des vitesses et pressions sensiblement plus élevées grâce à des corrélations basées sur un nombre important d'essais de validation sur bancs industriels.

Ces tribomètres conventionnels (comme, par exemple, connus du document FR1500941 A) présentent un certain nombre d'inconvénients :
- les éprouvettes utilisées peuvent être coûteuses dues à une forme complexe, certaines présentant une forme cylindrique avec un coin,
- la mise en oeuvre des essais peut s'avérer coûteuse,
- les conditions de fonctionnement peuvent être insuffisantes pour tester des matériaux à haute résistance à l'usure tels que les céramiques ou certains aciers,
- il est difficile, voire impossible, d'appliquer simultanément des vitesses et des pressions élevées, les vitesses étant généralement limitées à environ 0,1 m/s et les pressions de contact maximales étant de l'ordre du GPa,
- il est difficile, voire impossible, de contrôler à la fois la cinématique du contact et la cinétique de la charge au niveau des contacts.

L'invention vise à remédier à tout ou partie des inconvénients précités.

L'invention a pour objet un tribomètre comportant :
- un premier ensemble de support agencé pour recevoir une éprouvette centrale, laquelle est cylindrique de révolution, et l'entraîner en rotation suivant son axe,
- un second ensemble de support agencé pour recevoir une pluralité d'éprouvettes périphériques, de préférence trois, et permettre le contact de ladite éprouvette centrale simultanément avec lesdites éprouvettes périphériques dans une configuration isostatique, de sorte que l'éprouvette centrale puisse, en étant entraînée en rotation, frotter contre les éprouvettes périphériques.

Grâce à l'invention, il est possible de mener des tests avec des éprouvettes présentant une forme simple et donc un coût de fabrication réduit.

De préférence, l'une au moins des trois éprouvettes périphériques, notamment toutes les trois, présentent chacune une forme choisie de manière à permettre un contact linéique avec l'éprouvette centrale, notamment suivant un segment de droite. Ceci permet notamment de générer des pertes de matière accrues et d'avoir des surfaces d'usure relativement importantes pouvant servir à caractériser les transformations chimiques de ces surfaces suite aux sollicitations tribologiques.

L'une au moins des trois éprouvettes périphériques, notamment toutes les trois, peuvent comporter chacune une face plane sur laquelle vient frotter l'éprouvette centrale, et peuvent présenter par exemple chacune une forme de plaquette.

Les éprouvettes peuvent ainsi avoir une forme particulièrement simple, ce qui permet de réduire de manière importante leur coût de fabrication. Ceci est particulièrement avantageux pour les matériaux céramiques tels que le carbure de tungstène ou l'alumine.

Le deuxième ensemble de support est de préférence agencé de sorte que les contacts des éprouvettes périphériques sur l'éprouvette centrale soient équiangulairement répartis autour de ladite éprouvette centrale, étant sensiblement à 120° l'un de l'autre, le contact entre, d'une part, l'éprouvette centrale et, d'autre part, les trois éprouvettes périphériques étant isostatique.

En entraînant l'éprouvette centrale à une vitesse de rotation élevée et/ou en augmentant le diamètre de cette éprouvette centrale, il est possible, grâce à l'invention, d'atteindre des vitesses de frottement de glissement très élevées, supérieures notamment à 1 m/s, pouvant notamment atteindre jusqu'à 15 m/s. Le contact isostatique précité peut encore permettre d'atteindre des pressions de contact très élevées, notamment supérieures à 1 GPa, par exemple jusqu'à 3 GPa.

Les contacts étant soumis à des sollicitations tribologiques sévères, à l'instar de conditions de fonctionnement réelles, les essais sur bancs industriels peuvent être réduits, ce qui permet de limiter les coûts de développement.

L'invention permet en outre de faciliter la formulation de lubrifiants (liquide, solide, multiphasique, émulsion, dispersion, lubrifiant aqueux, lubrifiant solide, huile, graisse) et l'élaboration de matériaux pour des systèmes mécaniques sollicités dans des conditions de pressions, vitesses de glissement et température de contact très élevées.

L'invention est particulièrement bien adaptée aux tests avec des matériaux métalliques et céramiques très résistants à l'usure, tels que le carbure de tungstène, dont l'usure ne se produit qu'à partir de seuils d'énergie considérable, de l'ordre de 1 kJ/µm³.

L'invention permet encore :
- la réalisation de cartographies pour les coefficients de frottement/pression/vitesse, pour un couple matériau/lubrifiant donné, permettant ainsi d'affiner les conditions optimales de lubrification,
- l'étude des effets thermiques en lubrification en conditions de vitesses et pressions très élevées en exploitant des vitesses élevées couplées à une mesure en continu de la température moyenne de contact,
- l'étude, sur le plan fondamental, des processus d'usure et des mécanismes de lubrification, en conditions de vitesses et pressions extrêmes. Dans ces gammes de vitesses et de pressions, le comportement tribologique des lubrifiants et des surfaces qu'ils protègent est largement méconnu car il est actuellement difficile de simuler de telles conditions en dehors des bancs industriels.

Le deuxième ensemble de support peut comporter trois pièces de support portant chacune une éprouvette, l'une au moins de ces trois pièces de support pouvant être mobile par rapport aux deux autres.

Les trois pièces de support peuvent être agencées pour former une cavité, laquelle peut contenir un produit, notamment un lubrifiant, les éprouvettes centrale et périphériques pouvant s'étendre au moins partiellement dans cette cavité de sorte que les contacts de l'éprouvette centrale avec les éprouvettes périphériques peuvent, le cas échéant, être au moins partiellement noyés dans le produit.

Le tribomètre selon l'invention permet ainsi de tester des contacts secs ou lubrifiés.

Le tribomètre peut comporter un circuit agencé pour établir une circulation de produit, notamment de lubrifiant, dans la cavité.

Cette circulation peut permettre le contrôle de la température dans la cavité.

Le tribomètre peut comporter un dispositif d'application de charge agencé pour appliquer une force sur l'une au moins des pièces de support, notamment sur chacune des trois.

Le dispositif d'application de charge peut comporter trois poussoirs agencés pour exercer sur chacune des pièces de support respectivement, une force sensiblement normale à l'axe de rotation de l'éprouvette centrale.

Chaque poussoir peut être entraîné par un actionneur comportant un bras articulé, solidaire à une extrémité, d'une partie fixe du tribomètre et, à l'autre extrémité, d'un organe d'entraînement axialement mobile.

Le tribomètre comporte avantageusement un dispositif de répartition de charge agencé pour commander le dispositif d'application de charge, notamment en contrôlant le déplacement des organes d'entraînement du dispositif d'application de charge.

Chaque organe d'entraînement est avantageusement solidaire d'une poulie d'entraînement et le dispositif de répartition de charge peut comporter une courroie engagée sur les trois poulies des organes d'entraînement, le dispositif de répartition de charge étant en outre agencé pour exercer sur la courroie une tension variable.

Le tribomètre selon l'invention permet de réguler en temps réel la charge appliquée sur les éprouvettes.

Le dispositif de répartition de charge peut comporter deux poulies de guidage de la courroie disposées chacune entre deux poulies d'entraînement.

Le dispositif de répartition de charge peut encore comporter deux poulies de charge sur lesquelles sont fixées respectivement les deux extrémités de la courroie.

L'une au moins des poulies de charge peut être entraînée en rotation à l'aide d'une bande fixée à une extrémité à cette poulie de charge et à l'autre extrémité à un chariot mobile.

Le dispositif de répartition de charge peut comporter une première membrane souple agencée pour entraîner le chariot mobile en déplacement, en fonction de la pression dans la première membrane.

Dans un exemple de mise en oeuvre de l'invention, le tribomètre comporte un système de régulation en pression agencé pour contrôler la pression de la première membrane, ce système comportant par exemple un ressort ayant une première extrémité apte à être déplacée par l'intermédiaire d'un moteur et une deuxième extrémité solidaire d'un bras agencé pour agir sur une deuxième membrane souple en communication avec la première membrane souple.

Le tribomètre selon l'invention permet ainsi le contrôle simultané de la cinétique de chargement et de la cinématique des contacts.

Le tribomètre peut être agencé pour permettre d'atteindre une charge maximale de 2.10⁴ N, par poussoir, en 4 s, soit un gradient de pression maximale de l'ordre de 7000 bars par seconde.

Les dispositifs d'application et de répartition de charge et le système de régulation en pression sont extérieurs à la cavité précitée et peuvent ne jamais être en contact direct avec le lubrifiant.

Le tribomètre peut être agencé pour agir sur la position de l'une au moins des pièces de support en cas de déplacement d'un contact entre l'éprouvette centrale et l'une des éprouvettes périphériques.

Le dispositif de charge peut être associé à un capteur de force tangentielle apte à mesurer la force tangentielle au niveau d'un contact entre l'éprouvette centrale et l'une des éprouvettes périphériques.

La mesure en continu du couple du moteur entraînant l'éprouvette centrale en rotation permet une évaluation précise des efforts de frottement sur les deux autres contacts, non associés à un capteur de force tangentielle.

Le tribomètre peut comporter trois capteurs de force normale agencés pour mesurer la force normale au niveau de chaque contact entre l'éprouvette centrale et les éprouvettes périphériques.

L'invention sera mieux comprise à la lecture de la description détaillée qui va suivre, d'un exemple de mise en oeuvre non limitatif de l'invention, et à l'examen du dessin annexé, sur lequel :
- la figure 1 représente, schématiquement et partiellement, en perspective, un tribomètre conforme à l'invention,
- la figure 2 est une vue, schématique et partielle, des contacts entre les éprouvettes,
- la figure 3 représente, schématiquement et partiellement, le second ensemble de support du tribomètre de la figure 1,
- la figure 4 représente, schématiquement et partiellement, le dispositif d'application de charge du tribomètre,
- la figure 5 représente, schématiquement et partiellement, en perspective, le dispositif de répartition de charge du tribomètre,
- la figure 6 est une vue en perspective, schématique et partielle, du dispositif d'application de charge du tribomètre,
- la figure 7 représente, schématiquement et partiellement, de manière isolée, un chariot mobile du dispositif de charge,
- la figure 8 est une vue schématisant les éléments du dispositif de répartition de charge du tribomètre,
- la figure 9 représente, schématiquement et partiellement, un système de régulation en pression du tribomètre, et
- la figure 10 est une vue schématisant le système de régulation en pression du tribomètre.

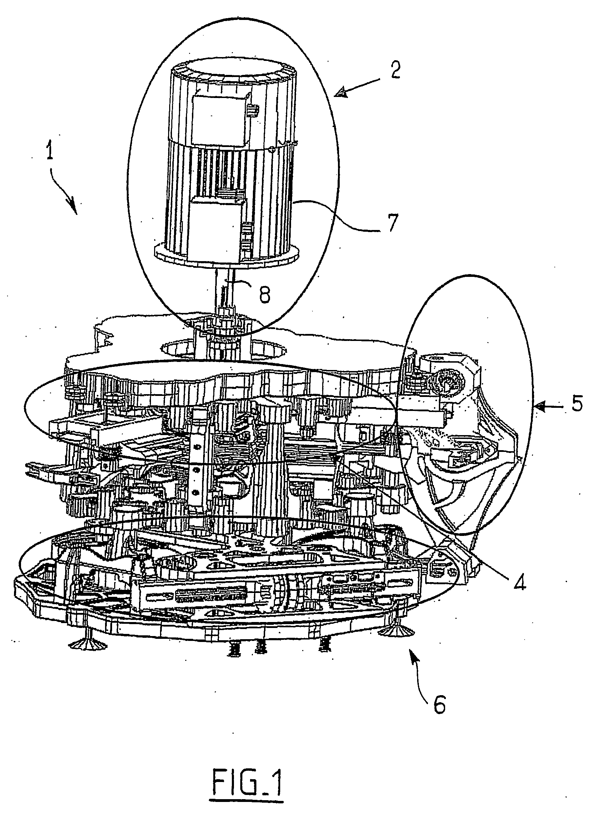

On a représenté sur la figure 1 différents éléments constitutifs d'un tribomètre 1 conforme à l'invention.

Le tribomètre 1 comporte un premier ensemble de support 2, un deuxième ensemble de support 4, un dispositif d'application de charge 5, et un dispositif de répartition de charge 6.

Le premier ensemble de support 2 porte une éprouvette centrale 3 cylindrique de révolution.

L'éprouvette centrale 3 est fixée à un arbre d'axe X d'un moteur d'entraînement 7 par l'intermédiaire d'une broche 8 agencée pour permettre la mise en place d'éprouvettes dont le diamètre peut varier de 8 mm à 32 mm, dans l'exemple considéré.

Le moteur 7 est agencé pour entraîner l'éprouvette 3 en rotation à une vitesse pouvant aller jusqu'à 9000 tr/mn, ce qui permet d'atteindre des vitesses linéaires allant environ de 3,8 m/s à 15 m/s.

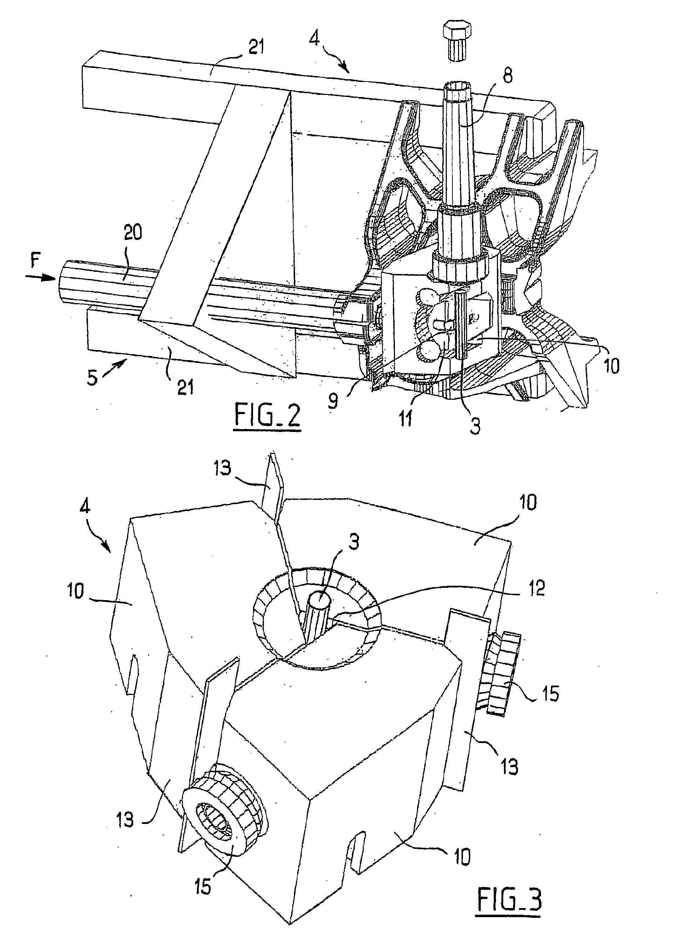

Le deuxième ensemble de support 4 comporte trois pièces de support 10, comme on peut le voir sur les figures 2 et 3.

Chaque pièce de support 10 porte une éprouvette périphérique 9, se présentant sous la forme d'une plaquette, par exemple en inox, celle-ci étant insérée dans un renfoncement de la pièce de support 10 et y étant maintenue, dans l'exemple considéré, par encliquetage.

Chaque pièce de support 10 présente un évidement 11 apte à définir avec les deux évidements 11 des deux autres pièces de support 10, lorsque les trois pièces 10 sont réunies comme illustré sur la figure 3, une cavité 12 dans laquelle sont logées les trois éprouvettes périphériques 9 et dans laquelle peut s'engager l'éprouvette centrale 3 lorsque le tribomètre est en marche.

Des joints d'étanchéité 13 peuvent être interposés chacun entre deux pièces de support 10, comme on peut le voir sur la figure 3, afin de rendre étanche la cavité 12.

La cavité 12 peut servir de réservoir pour un lubrifiant, notamment liquide, dans lequel sont noyés les contacts entre l'éprouvette centrale 3 et les éprouvettes périphériques 9.

Cette cavité 12 peut être mise en communication avec un circuit agencé pour générer une circulation de produit, notamment d'un lubrifiant, dans la cavité 12 par l'intermédiaire de deux orifices respectivement d'entrée et de sortie 15, réalisées sur deux des pièces de support 10.

Le dispositif d'application de charge 5 comporte trois poussoirs 20 aptes chacun à exercer une force normale F sur une pièce de support 10, comme illustré sur la figure 2.

En appliquant une force F très élevée (jusqu'à environ 2.10⁴ N), il est possible d'atteindre des pressions de contact très élevées (jusqu'à environ 3 GPa).

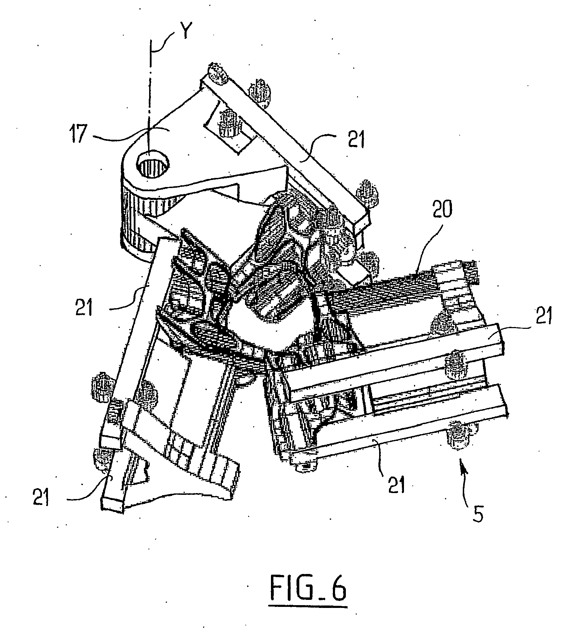

Chaque poussoir 20 est monté coulissant sur des rails 21, comme on peut le voir sur les figures 2 et 6.

Par souci de clarté, un seul poussoir 20, parmi trois, est représenté sur la figure 6.

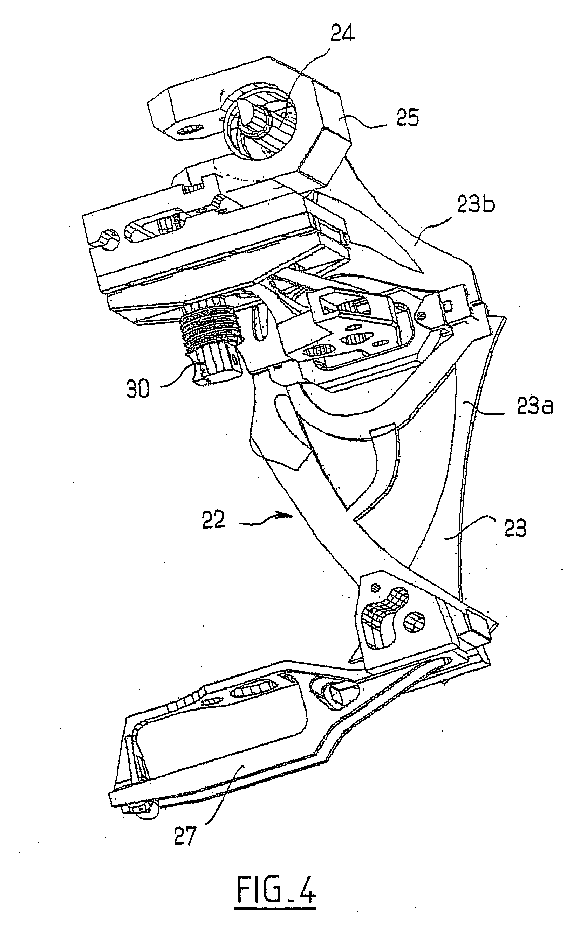

Chaque poussoir 20 est commandé en déplacement par un actionneur 22 représenté sur la figure 4.

Cet actionneur 22 comporte un bras pivotant 23, lequel comprend une partie inférieure 23a et une partie supérieure 23b articulées entre elles.

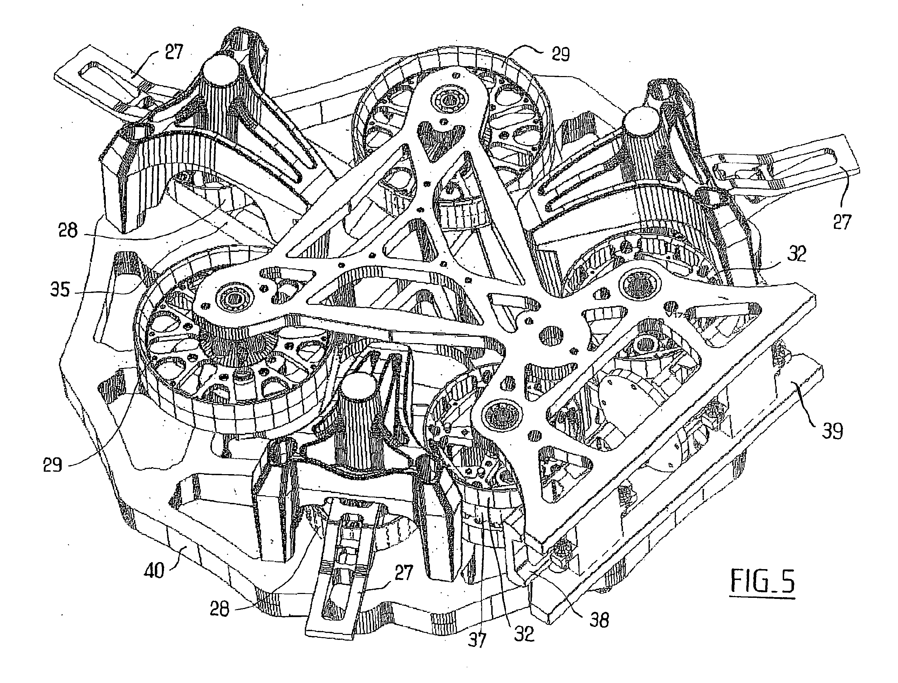

La partie inférieure 23a du bras 23 est articulée sur un organe d'entraînement 27 monté à coulissement sur un châssis 40 du tribomètre 1, comme on peut le voir notamment sur la figure 5.

La partie supérieure 23b du bras 23 est articulée sur une partie fixe 25 du tribomètre.

L'actionneur 22 comporte un élément de rappel élastique, notamment un ressort, non représenté, permettant de rappeler le bras pivotant 23 dans une position de repos. Le ressort permet en outre de filtrer des variations parasites de la charge.

Le dispositif de charge 5 comporte trois capteurs de force normale 30 disposés à proximité du poussoir 20 permettant de mesurer la force normale appliquée sur celui-ci et un capteur supplémentaire, non représenté, permettant de mesurer une force tangentielle sur l'un des contacts frottants.

Le capteur de force normale 30 est avantageusement placé après l'élément de rappel élastique précité.

Comme on peut le voir sur la figure 6, le dispositif de charge 5 comporte un ensemble 17 pivotant autour d'un axe Y et portant un poussoir 20, non représenté, pour appliquer une force normale sur une pièce de support 10.

L'ensemble 17 est associé au capteur supplémentaire précité, qui peut être constitué par un capteur de force normale, de manière à ce que lorsque l'ensemble 17 pivote autour de l'axe Y, celui-ci vient s'appliquer sur le capteur supplémentaire.

Ce capteur permet ainsi, en mesurant une force normale, de déterminer la force tangentielle sur l'un des contacts frottant.

De plus, le pivotement de l'ensemble 17 autour de l'axe Y permet de compenser un éventuel écart, lors du fonctionnement, entre les contacts frottants.

Chaque organe d'entraînement 27 porte une poulie d'entraînement 28, comme on peut le voir sur la figure 5.

Le châssis 40 porte d'une part deux poulies de guidage 29 disposées chacune entre deux poulies d'entraînement 28, et d'autre part deux poulies de charge 32.

Dans l'exemple considéré, les poulies d'entraînement 28 présentent un diamètre de 150 mm et les poulies de guidage 29 un diamètre de 250 mm.

Les poulies de charge 32 comportent une partie supérieure ayant un diamètre de 175 mm et une partie inférieure ayant un diamètre de 200 mm.

Une courroie 35, réalisée dans l'exemple décrit en Kevlar^{®}, est engagée sur les poulies d'entraînement 28 par l'extérieur, et sur les poulies de guidage 29 par l'intérieur, et est fixée à ses extrémités aux poulies de charge 32.

Chaque poulie de charge 32 est entraînée en rotation par une bande 37, notamment métallique, dont une extrémité est reliée à la partie inférieure de la poulie de charge 32 et l'autre extrémité à un chariot 38 monté à coulissement sur un support 39.

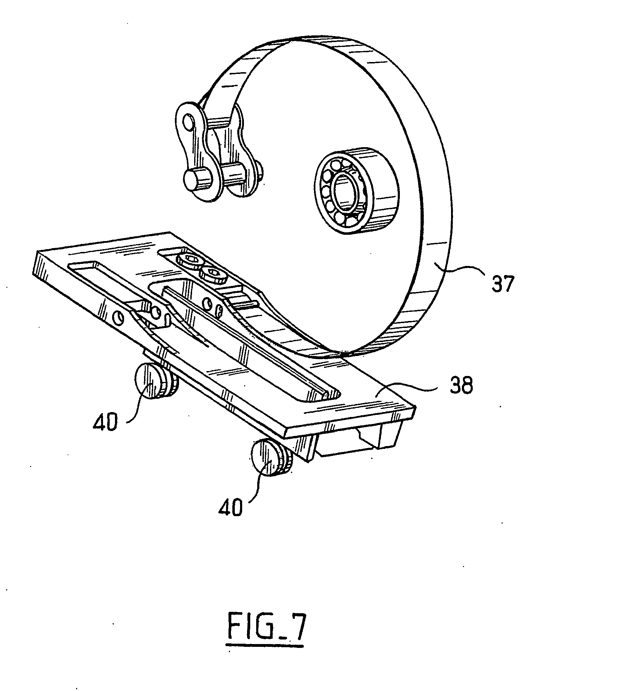

Chaque chariot 38, illustré sur la figure 7, comporte des galets 40 pouvant glisser sur le support 39.

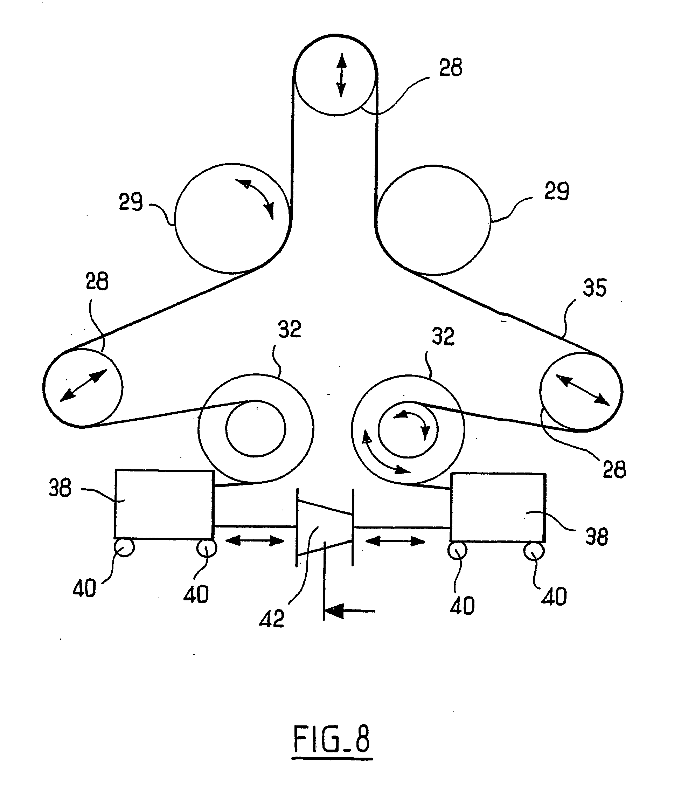

Comme on peut le voir sur le schéma de la figure 8, lorsque la courroie 35 est tendue par déplacement des chariots 38, celle-ci entraîne les trois poulies d'entraînement 28 vers l'intérieur de sorte que les actionneurs 22 entraînent les poussoirs 20 correspondants pour exercer une force prédéterminée sur les pièces de support 10.

Le déplacement des chariots 38 est commandé par la déformation d'une première membrane souple 42 disposée sur le support 39, entre les deux chariots 38.

La première membrane 42 est associée à un système de régulation en pression 50 permettant de contrôler la pression dans la première membrane 42.

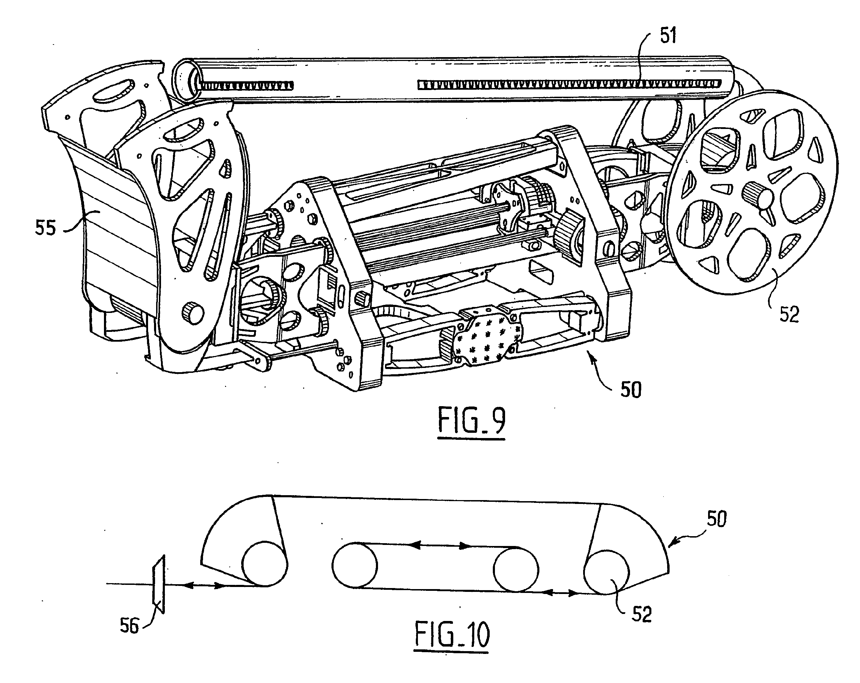

Comme on peut le voir sur la figure 9, ce système 50 comporte un ressort 51 relié à une extrémité à une poulie 52, laquelle est entraînée en rotation par un moteur, non représenté.

Le ressort 51 est solidaire à l'autre extrémité d'un bras 55 permettant de faire varier la pression dans une deuxième membrane souple 56, illustrée sur la figure 10, cette dernière étant en communication par l'intermédiaire d'une tubulure avec la première membrane souple 42.

Les mesures suivantes sont effectuées lors du fonctionnement du tribomètre :
- mesures de la force tangentielle dans une plage de 0 à 5000 N, mesurée à 0,08 N et régulée à 0,5 N sur un contact,
- mesures de la force normale sur chaque contact dans une plage de 0 à 20000 N, mesurée à 2 N et régulée à 1 N,
- mesure du couple moteur,
- mesure de la température de contact à 0,1 °C, et
- régulation et mesure de la température du lubrifiant dans le cas d'un contact lubrifié à 0,1 °C près.

Le tribomètre 1 permet une application et une régulation de la charge avec la possibilité de soumettre le contact à une cinétique de chargement contrôlée jusqu'à 2.10⁴ N tout en maîtrisant la cinématique du contact à vitesse élevée.

## Revendications

1. Tribomètre (1) comportant :
- un premier ensemble de support (2) agencé pour recevoir une éprouvette centrale (3), laquelle est cylindrique de révolution, et l'entraîner en rotation suivant son axe (X),
- un second ensemble de support (4) agencé pour recevoir trois éprouvettes périphériques et permettre le contact de ladite éprouvette centrale (3) simultanément avec lesdites éprouvettes périphériques (9), dans une configuration isostatique, de sorte que l'éprouvette centrale puisse, en étant entraînée en rotation, frotter contre les éprouvettes périphériques, le deuxième ensemble de support (4) comportant trois pièces de support (10) portant chacune une éprouvette (9) **caractérisé en ce que** chacune de ces trois pièces de support est mobile par rapport aux deux autres.

2. Tribomètre selon la revendication précédente, **caractérisé par le fait que** l'une au moins des trois éprouvettes périphériques (9), notamment toutes les trois, présentent chacune une forme choisie de manière à permettre un contact linéique avec l'éprouvette centrale, notamment suivant un segment de droite.

3. Dispositif selon l'une des deux revendications précédentes, **caractérisé par le fait que** l'une au moins des trois éprouvettes périphériques (9), notamment toutes les trois, comportent chacune une face plane sur laquelle vient frotter l'éprouvette centrale.

4. Tribomètre selon la revendication précédente, **caractérisé par le fait que** l'une au moins des trois éprouvettes périphériques (9), notamment toutes les trois, présentent chacune une forme de plaquette.

5. Tribomètre selon l'une quelconque des revendications précédentes, **caractérisé par le fait que** le deuxième ensemble de support (4) est agencé de sorte que les contacts des éprouvettes périphériques (9) sur l'éprouvette centrale (3) sont équiangulairement répartis autour de ladite éprouvette centrale.

6. Tribomètre selon l'une quelconque des revendications précédentes, **caractérisé par le fait que** les trois pièces de support (10) sont agencées pour former une cavité (12), laquelle peut contenir un produit, notamment un lubrifiant, les éprouvettes centrale et périphériques pouvant s'étendre au moins partiellement dans cette cavité de sorte que les contacts de l'éprouvette centrale sur les éprouvettes périphériques sont au moins partiellement noyés dans le produit.

7. Tribomètre selon la revendication précédente, **caractérisé par le fait qu'**il comporte un circuit agencé pour établir une circulation de produit dans la cavité.

8. Tribomètre selon l'une quelconque des revendications 5 à 7, **caractérisé par le fait qu'**il comporte un dispositif d'application de charge (5) agencé pour appliquer une force sur l'une au moins des pièces de support, notamment toutes les trois.

9. Tribomètre selon l'une quelconque des revendications précédentes, **caractérisé par le fait que** le dispositif d'application de charge (5) comporte trois poussoirs (20) agencés pour exercer sur chacune des pièces de support respectivement une force sensiblement normale à l'axe de rotation de l'éprouvette centrale, chaque poussoir étant entraîné par un actionneur (22), lequel comporte un bras articulé (23), solidaire à une extrémité d'une partie fixe (25) du tribomètre et, à l'autre extrémité, d'un organe d'entraînement mobile (27).

10. Tribomètre selon la revendication précédente, **caractérisé par le fait qu'**il comporte un dispositif de répartition de charge (6) pour commander le déplacement des organes d'entraînement.

11. Tribomètre selon la revendication 10, **caractérisé par le fait que** chaque organe d'entraînement est solidaire d'une poulie d'entraînement (18) et **par le fait que** le dispositif de répartition de charge comporte une courroie (35) engagée sur les trois poulies d'entraînement (28), le dispositif de répartition de charge étant agencé pour exercer sur la courroie une tension variable.

12. Tribomètre selon la revendication précédente, **caractérisé par le fait que** le dispositif de répartition de charge comporte deux poulies de guidage (29) de la courroie, disposées chacune entre deux poulies d'entraînement.

13. Tribomètre selon l'une des revendications 11 et 12, **caractérisé par le fait que** le dispositif de répartition de charge comporte deux poulies de charge (32) sur lesquelles sont fixées respectivement les deux extrémités de la courroie.

14. Tribomètre selon la revendication précédente, **caractérisé par le fait que** l'une au moins des poulies de charge (32) est entraînée en rotation à l'aide d'une bande (37) fixée à une extrémité à cette poulie de charge (32) et à l'autre extrémité à un chariot mobile (38).

15. Tribomètre selon la revendication précédente, **caractérisé par le fait que** le dispositif de répartition de charge comporte une première membrane (42) agencée pour entraîner le chariot mobile (3 8) en déplacement.

16. Tribomètre selon la revendication précédente, **caractérisé par le fait qu'**il comporte un système de régulation en pression agencé pour contrôler la pression de la première membrane, le système comportant par exemple un ressort ayant une première extrémité apte à être déplacée par l'intermédiaire d'un moteur et une extrémité solidaire d'un bras agencé pour agir sur une deuxième membrane souple en communication avec la première.

## Claims

1. A tribometer (1) comprising:
a first support assembly (2) configured to receive a central test piece (3), which test piece is circularly cylindrical, and to drive it in rotation about its axis (X) ; and
a second support assembly (4) configured to receive three peripheral test pieces and to enable said central test piece (3) to come simultaneously into contact with said three peripheral test pieces (9) in an isostatic configuration, such that while being driven in rotation the central test piece can rub against the peripheral test pieces, the second support assembly (4) comprising three support parts (10) each carrying a respective peripheral test piece (9), each of these three support parts is movable relative to the other two.

2. A tribometer according to the preceding claim, **characterized by** the fact that at least one of the three peripheral test pieces (9), and in particular all three of them, presents a shape selected in such a manner as to enable contact with the central test piece to be linear, in particular along a straight line segment.

3. A device according to either one of the two preceding claims, **characterized by** the fact that at least one of the three peripheral test pieces (9), and in particular all three of them, comprises a plane face whereby it rubs against the central test piece.

4. A tribometer according to the preceding claim, **characterized by** the fact that at least one of the three peripheral test pieces (9), and in particular all three of them, presents the form of a plate.

5. A tribometer according to any preceding claim, **characterized by** the fact that the second support assembly (4) is configured in such a manner that the contacts between the peripheral test pieces (9) and the central test piece (3) are distributed at equal angles around said central test piece.

6. A tribometer according to any preceding claim, **characterized by** the fact that the three support parts (10) are configured to form a cavity (12) suitable for containing a fluid, in particular a lubricant, the central and peripheral test pieces extending at least in part in said cavity such that the contacts between the central test piece and the peripheral test pieces are immersed at least in part in the fluid.

7. A tribometer according to the preceding claim, **characterized by** the fact that it comprises a circuit configured to establish circulation of the fluid in the cavity.

8. A tribometer according to any one of claims 5 to 7, **characterized by** the fact that it comprises a load-application device (5) configured to apply a force on at least one of the support parts, and in particular on all three of them.

9. A tribometer according to any preceding claim, **characterized by** the fact that the load-application device (5) comprises three pushers (20) configured to exert on each of the respective support parts a force that is substantially normal to the axis of rotation of the central test piece, each pusher being driven by an actuator (22) comprising a hinged arm (23) secured at one end to a stationary portion (25) of the tribometer, and at its other end to a moving drive member (27).

10. A tribometer according to the preceding claim, **characterized by** the fact that it comprises a load-sharing device (6) for controlling the displacement of the drive members.

11. A tribometer according to claim 10, **characterized by** the fact that each drive member is secured to a drive pulley (18), and by the fact that the load-sharing device comprises a belt (35) engaged on the three drive pulleys (28), the load-sharing device being configured to exert variable tension on the belt.

12. A tribometer according to the preceding claim, **characterized by** the fact that the load-sharing device comprises two guide pulleys (29) for guiding the belt, each being disposed between two drive pulleys.

13. A tribometer according to claim 11 or claim 12, **characterized by** the fact that the load-sharing device comprises two load pulleys (32) having the two ends of the belt secured respectively thereto.

14. A tribometer according to the preceding claim, **characterized by** the fact that at least one of the load pulleys (32) is driven in rotation by a strip (37) secured at one end to said load pulley (32) and at its other end to a moving carriage (38).

15. A tribometer according to the preceding claim, **characterized by** the fact that the load-sharing device comprises a first membrane (42) configured to drive displacement of the moving carriage (38).

16. A tribometer according to the preceding claim, **characterized by** the fact that it comprises a pressure-regulator system configured to control the pressure of the first membrane, the system comprising, for example, a spring having a first end suitable for being moved by a motor and a second end secured to an arm configured to act on a second flexible membrane in communication with the first.

## Patentansprüche

1. Tribometer (1) mit:
- einer ersten Trägeranordnung (2), die eingerichtet ist, um ein zentrales Probestück (3) aufzunehmen, welches rotationszylindrisch ist, und es gemäß seiner Achse (X) in Rotation zu versetzen,
- einer zweiten Trägeranordnung (4), die eingerichtet ist, um drei periphere Probestücke aufzunehmen und den Kontakt des zentralen Probestücks (3) gleichzeitig mit den peripheren Probestücken (9) in einer isostatischen Konfiguration zu ermöglichen, so daß das in Rotation versetzte zentrale Probestück gegen die peripheren Probestücke reiben kann, wobei die zweite Trägeranordnung (4) drei Trägerstücke (10) aufweist, die jeweils ein Probestück (9) tragen,
**dadurch gekennzeichnet, daß** jedes der drei Trägerstücke bezüglich den zwei anderen bewegbar ist.

2. Tribometer nach dem vorstehenden Anspruch, **dadurch gekennzeichnet, daß** mindestens eines der drei peripheren Probestücke (9), insbesondere alle drei, jeweils eine Form aufweisen, die so gewählt ist, um einen längenbezogenen Kontakt, insbesondere entlang eines geraden Segments, mit dem zentralen Probestück zu erlauben.

3. Vorrichtung nach einem der zwei vorstehenden Ansprüche, **dadurch gekennzeichnet, daß** mindestens eines der drei peripheren Probestücke (9), insbesondere alle drei, jeweils eine ebene Fläche aufweisen, an welcher das zentrale Probestück zur Reibung kommt.

4. Tribometer nach dem vorstehenden Anspruch, **dadurch gekennzeichnet, daß** mindestens eines der drei peripheren Probestücke (9), insbesondere alle drei, jeweils eine Plattenform aufweisen.

5. Tribometer nach einem der vorstehenden Ansprüche, **dadurch gekennzeichnet, daß** die zweite Trägeranordnung (4) so eingerichtet ist, daß die Kontakte der peripheren Probestücke (9) an dem zentralen Probestück (3) im gleichen Winkel um das zentrale Probestück herum verteilt sind.

6. Tribometer nach einem der vorstehenden Ansprüche, **dadurch gekennzeichnet, daß** die drei Trägerstücke (10) eingerichtet sind, um einen Hohlraum (12) zu bilden, welcher ein Produkt, insbesondere ein Schmiermittel, enthalten kann, wobei sich das zentrale und die peripheren Probestücke mindestens teilweise in diesen Hohlraum hinein erstrecken, so daß die Kontakte des zentralen Probestücks an den peripheren Probestücken mindestens teilweise in dem Produkt eingetaucht sind.

7. Tribometer nach dem vorstehenden Anspruch, **dadurch gekennzeichnet, daß** es einen Kreislauf aufweist, der eingerichtet ist, um eine Zirkulation des Produkts in dem Hohlraum bereitzustellen.

8. Tribometer nach einem der Ansprüche 5 bis 7, **dadurch gekennzeichnet, daß** es eine Lastanlegevorrichtung (5) aufweist, die eingerichtet ist, um eine Kraft auf mindestens eines der Trägerstücke, insbesondere auf alle drei, auszuüben.

9. Tribometer nach einem der vorstehenden Ansprüche, **dadurch gekennzeichnet, daß** die Lastanlegevorrichtung (5) drei Drücker (20) aufweist, die eingerichtet sind, um auf jedes der Trägerstücke jeweils eine zur Rotationsachse des zentralen Probestücks im wesentlichen senkrechte Kraft auszuüben, wobei jeder Drücker von einem Betätigungselement (22) angetrieben wird, das einen Gelenkarm (23) aufweist, der an einem Ende mit einem festen Teil (25) des Tribometers verbunden ist und an dem anderen Ende mit einem beweglichen Antriebselement (27) verbunden ist.

10. Tribometer nach dem vorstehenden Anspruch, **dadurch gekennzeichnet, daß** es eine Lastverteilungsvorrichtung (6) aufweist, um die Verschiebung der Antriebselemente zu steuern.

11. Tribometer nach Anspruch 10, **dadurch gekennzeichnet, daß** jedes Antriebselement mit einer Antriebsrolle (28) verbunden ist, und **dadurch**, daß die Lastverteilungsvorrichtung einen Riemen (35) aufweist, der mit den drei Antriebsrollen (28) im Eingriff ist, wobei die Lastverteilungsvorrichtung eingerichtet ist, um auf den Riemen eine variable Spannung auszuüben.

12. Tribometer nach dem vorstehenden Anspruch, **dadurch gekennzeichnet, daß** die Lastverteilungsvorrichtung zwei Führungsrollen (29) zum Führen des Riemens aufweist, die jeweils zwischen zwei Antriebsrollen angeordnet sind.

13. Tribometer nach einem der Ansprüche 11 und 12, **dadurch gekennzeichnet, daß** die Lastverteilungsvorrichtung zwei Lastrollen (32) aufweist, an welchen jeweils die zwei Enden des Riemens angebracht sind.

14. Tribometer nach dem vorstehenden Anspruch, **dadurch gekennzeichnet, daß** mindestens eine der Lastrollen (32) mit Hilfe eines Bandes (37), das an einem Ende an dieser Lastrolle (32) angebracht ist und an dem anderen Ende an einem beweglichen Wagen (38) angebracht ist, in Rotation versetzt wird.

15. Tribometer nach dem vorstehenden Anspruch, **dadurch gekennzeichnet, daß** die Lastverteilungsvorrichtung eine erste Membran (42) aufweist, die eingerichtet ist, um den beweglichen Wagen (38) in Verschiebung zu versetzen.

16. Tribometer nach dem vorstehenden Anspruch, **dadurch gekennzeichnet, daß** es ein Druckregelungssystem aufweist, das eingerichtet ist, um den Druck der ersten Membran zu steuern, wobei das System zum Beispiel eine Feder aufweist, die ein erstes Ende hat, das mit Hilfe eines Motors verschoben werden kann, und ein Ende hat, das mit einem Arm verbunden ist, der eingerichtet ist, um auf eine weiche zweite Membran einzuwirken, die in Verbindung mit der ersten steht.
